# EUROPEAN PATENT SPECIFICATION

(11) **EP 2 121 101 B1**
(45) Date of publication and mention of the grant of the patent: **24.08.2016**
(21) Application number: 08729581.2
(22) Date of filing: 12.02.2008
(51) Int. Cl.: A61M 25/09

(54) **DUAL STIFFNESS WIRE GUIDE**
FÜHRUNGSDRAHT MIT DOPPELTER VERSTEIFUNG
GUIDE-FIL À DOUBLE RAIDEUR

(30) Priority: 15.02.2007 US 901408 P
(43) Date of publication of application: 25.11.2009
(73) Proprietor: Cook Medical Technologies LLC, Bloomington, IN 47404 (US)
(72) Inventor: BUNCH, Tyler, J., Bloomington, IN 47404 (US); RYAN, Walter, N., Greenville, NC 27858 (US); EELLS, Scott, E., Bloomington, IN 47404 (US)
(74) Representative: Garratt, Peter Douglas
(86) International application number: PCT/US2008/053636
(87) International publication number: WO 2008/100877

(56) References cited:
- EP-A- 1 325 763
- EP-A- 1 464 358
- WO-A-2005/007033
- WO-A-2006/002199
- US-A1- 2002 087 099
- US-A1- 2003 216 668
- US-A1- 2004 106 878
- US-A1- 2004 167 437

## Description

### TECHNICAL FIELD

The technical field of the invention is endoscopic and laparoscopic medical devices, and in particular, wire guides used in endoscopic and laparoscopic procedures.

### BACKGROUND

Wire guides are used extensively in what is referred to as the "Seldinger technique." In this technique, a physician guides a slim and flexible wire guide through a body lumen, such as a urethra, until the wire guide enters the body organ or cavity of interest. A wire guide is typically very narrow having a diameter from 0.018" (about .46 mm) to 0.038" (about 0.93 mm). A narrow wire guide with a lubricious coating, such as a hydrophilic coating, can pass through a body lumen with relative ease and comfort for the patient. Once the wire guide has been placed, the physician may then use the wire guide to pass larger instruments into the patient. For instance, the proximal end of the wire guide may be placed in a lumen of a catheter or an endoscope, and the catheter or endoscope may then also be guided into the patient along the same guide wire that was previously placed. An entire range of additional instruments, used for diagnostic or therapeutic procedures may then be used to help the patient.

Procedures involving a wire guide are certainly beneficial to the patient, because they offer an alternative to traditional open-cavity surgery. The trauma to the patient is greatly reduced, operating time is typically also greatly reduced, and the severity and length of the recovery procedure is likewise greatly reduced. Because of these advantages, wire guides are used extensively and opportunities for their improvement have arisen.

In particular, physicians want wire guides that are relatively stiff, and thus kink-resistant, but the wire guides should also be very flexible so that they may easily be guided to the desired location in the patient. To meet these needs, a variety of schemes have been tried to introduce both stiffness and flexibility in a wire guide. For instance, U.S. Patent Application Publication No. 2001/0021831 teaches the use of two wire guides, one wire guide helically wound within another, and each wire guide preferably multifilar. After the coils are wound, the inner coil is "unwound" so that it forms an interference fit with the outer coil. The resulting wire guide is stiff and kink-resistant, and has a high torque transfer capability. However, it is also relatively stiff and is not very flexible.

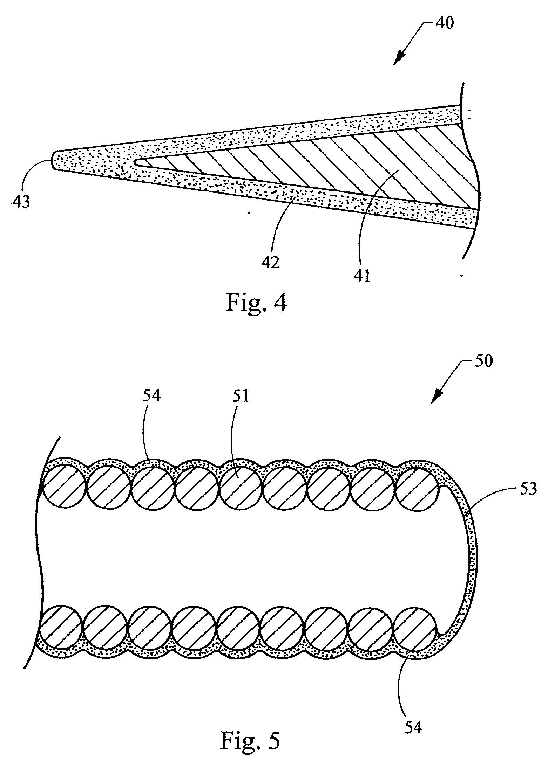

Another wire guide that attempts to blend stiffness with flexibility is described in U.S. Patent No. 6,001,068. This patent describes a relatively stiff proximal wire guide joined to a relatively flexible distal wire guide with a connector. However, as seen in Fig. 5 of the patent, there is a discontinuity of stiffness in the joint or connection between the two. This may lead to stress concentration, difficulty in achieving the desired performance of the wire guide, and could conceivably lead to kinking or failure of the wire guide. What is needed is a wire guide with better continuity between its distal and proximal portions.

A wire guide as defined in the preamble of claim 1 is disclosed in US2004/0106878.

### BRIEF SUMMARY

One embodiment is a composite wire guide. The composite wire guide includes a flexible distal portion, a stiff proximal portion, and a tapered connector joining the distal and proximal portions. Another embodiment is a composite wire guide. The composite wire guide includes a flexible distal portion made from a shape memory alloy, a stiff proximal portion, and a tapered connector with at least one helical or circumferential slit, the tapered connector adapted for joining the distal and proximal portions in a laser weld.

Another embodiment is a composite wire guide. The composite wire guide includes a central portion, a first flexible distal portion made from a shape memory alloy and connected to the central portion, and a second flexible distal portion connected to the central portion, wherein at least one of the first and second flexible distal portions are connected to the central portion by a tapered connector.

Another embodiment is a method for making a composite wire guide. The method includes furnishing a stiff proximal wire guide, connecting the stiff proximal wire guide to a tapered connector, and connecting the tapered connector to a flexible distal wire guide made from a shape memory alloy. There are many embodiments of the invention, of which the following descriptions and drawings show only a few. These descriptions are meant to be illustrative rather than limiting of the embodiments of the invention.

### BRIEF DESCRIPTION OF THE DRAWINGS

Fig. 1 is a cross sectional view of two wire guides of different stiffness joined by a first connector embodiment;
Fig. 2 is a cross sectional view of two wire guides of different stiffness joined by a second embodiment of a connector;
Fig. 3 is a plan view with a partial cross-section of a composite wire including wire guides of different stiffnesses; Fig. 3a depicts details from Fig. 3;
Fig. 4 is a cross sectional view of a first embodiment of one of the distal wire guides of Fig. 3;
Fig. 5 is a cross sectional view of a second embodiment of one of the distal wire guides of Fig. 3;
Figs. 6-10 depict alternative embodiments of flexible guide wires that may be used in composite guide wire embodiments; and
Figs. 11-18A depict alternative joint configurations that may be used in joining wire guides.
Fig. 19 is a cross sectional view of another embodiment of a wire guide.
Fig. 20 is an exploded view of the wire guide of Fig. 19.
Fig. 21 is the exploded view of the Fig. 20 with the connection portion in an expanded state.

### DETAILED DESCRIPTION OF THE DRAWINGS AND THE PRESENTLY PREFERRED EMBODIMENTS

Wire guides, also known as guide wires, have become indispensable in modern day surgery, especially in laparoscopic or endoscopic procedures. Although each surgeon has his or her own preferences, it is important that the wire guide have just the right "feel" that makes the surgeon comfortable in manipulating and using the wire guide. Accordingly, composite wire guide embodiments are described herein.

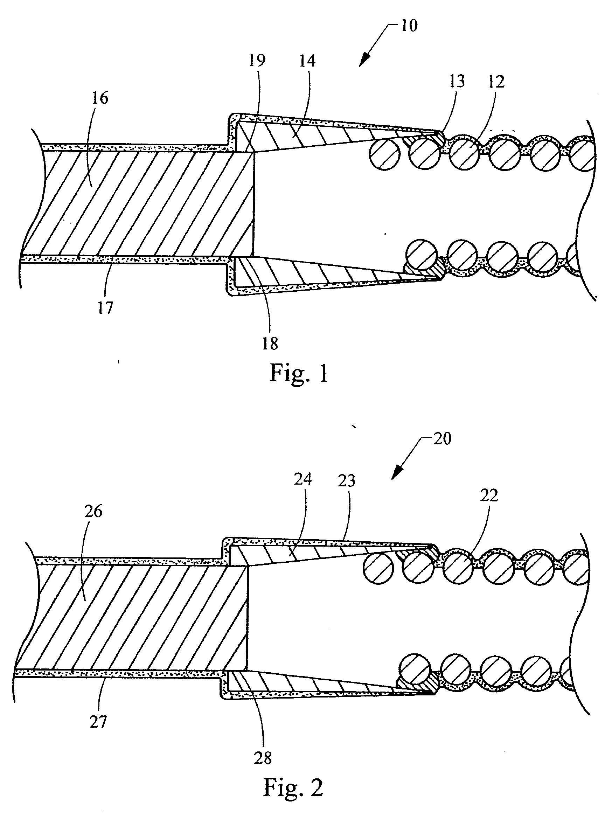

Fig. 1 depicts in cross-section a first embodiment 10 of a composite wire guide that includes a first wire guide 12, a connector 14, and a second wire guide 16. In this embodiment, wire guide 12 may be a stiffer, Amplatz type wire guide, made from coiled wire and preferably including an outer coating of Teflon or other lubricious substance. Hydrophilic coatings may also be used. In some embodiments, the hydrophilic coating is activated by immersing the desired end portion in saline solution or in water. This makes the immersed portion very slick and lubricious, and the insertion process is much easier for the surgeon and less traumatic for the patient.

Second wire guide 16 may be a looser, more flexible wire guide, such as the Hi-Wire^{®} wire guides available from Cook Urological Incorporated, Spencer, Indiana. Wire guide 16 may also include a lubricious coating 17 to make the guide wire easier to insert. The coating may be a hydrophilic coating, or a plastic coating such as PTFE or other plastic coating, or both. There is preferably a connector 14 that joins the two wire guides at a joint 18.

Connector 14 is tapered and is preferably made from nickel or a nickel alloy. Suitable nickel alloys include almost any alloy with a high nickel content, such as AMS 5806 (38 % Ni), AMS 5802 (38 % Ni), AMS 546 (53 % Ni), or the AMS 5600 series (generally 60% + Ni content). Almost any of the Nichrome, Inconel, Hastelloy, or Waspaloy alloys will also work well. Connectors made from other materials may also be used. However, since the end guide wire is typically made from Nitinol (nickel-titanium) shape memory alloy, connectors made from nickel work particularly well. Connectors made from titanium would probably also work well, but titanium connectors would be much more expensive than those made from a nickel alloy. Stainless steel connectors or connectors made from any medically-acceptable material would also work well, because typically the stiffer wire guide, such as an Amplatz-type, is made from less-expensive stainless steel.

The wire guides are joined to the connected at welds 13, 19. In order to join the wire guides at the connector, only laser welding is needed if the alloys are compatible, as described above. No small ring of solder or wire is needed in these cases. If incompatible alloys are used, a process that uses a small amount of solder, braze material, or even welding rod material may be used. Later, the joint may be ground or polished and is preferably coated with the above-mentioned lubricious coating, such as a plastic coating 14, a hydrophilic coating, or both.

Another embodiment is depicted in cross-section in Fig. 2. Composite wire guide 20 includes a stiff Amplatz-type wire guide 22 with coating 27. The coating may be a hydrophilic coating, or a plastic coating such as PTFE or other plastic coating, or both. Connector 24 is tapered on the inside only, and is also joined via weld 28 to a more flexible wire guide 26. Coating 23, 27 covers at least wire guide 26 and connector 24. Tapering of connector 14 or 24 assists in the transition from the stiffer wire guide to the more flexible wire guide. It is believed that the transition leads to less stress, less stress fractures, and a more reliable weld joint between the wire guides. This would also hold true if a braze or solder joint is used instead of the preferred laser weld.

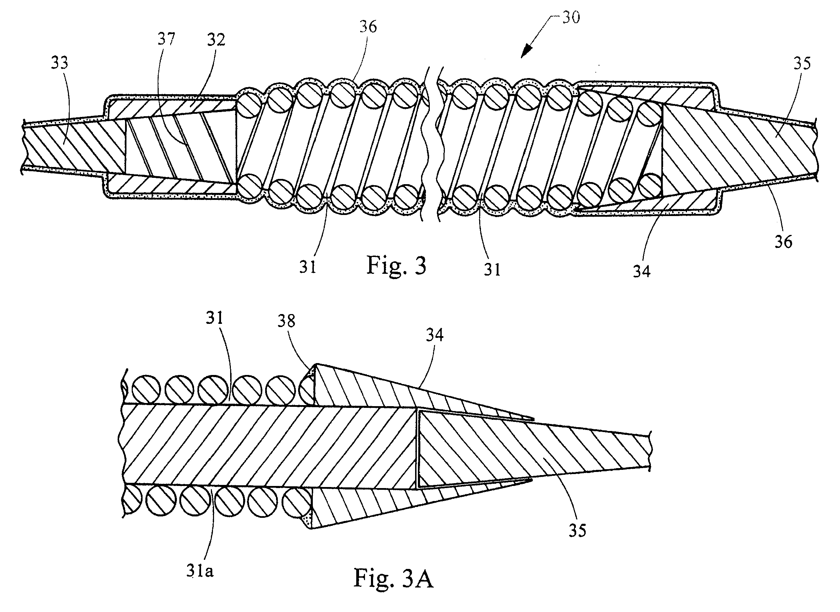

In addition to the composite guide wires described above, embodiments may also include guide wires with a stiffer center portion, such as an Amplatz, and a more flexible guide wire on either side of the central portion. One such embodiment is depicted in Figs. 3 and 3a. Composite wire guide 30 includes a stiff center portion 31, tapered connector 32, and a first flexible guide wire 33 connected by tapered connector 32. Tapered connector 32 may include one or more helical slits 37, which are preferably machined or laser-cut into the connector. The slits lend additional flexibility to the connector, which may more easily make the transition to flexible guide wire 33. Alternatively, the slits may be partially circumferential, so that connector integrity is retained. There is also a second flexible guide wire 35 connected at a second connector 34. Connector 34 is also preferably tapered. Composite wire guide 30 is preferably coated with coating 36, which may be a plastic, lubricious coating, and which may also include a hydrophilic coating.

Tapered connector 32 may be as described above and the composite wire guide may include a second tapered connector 34. As shown in Fig. 3a, second flexible wire guide 35 may have its proximal end tapered and then slid into connector 34. The center stainless steel portion 31 a of stiffer wire guide 31 may be ground to butt against wire guide 35 and placed adjacent. The wire guides and connector 34 may be secured to the joint by laser weld 38. Alternatively, a braze or solder joint may be made. The joint is then preferably ground or smoothed, and at least one coating applied. The coating is preferably a smooth plastic lubricious coating or a hydrophilic coating.

The ends of either of the flexible wire guides may be made as flexible as desired. Two embodiments are depicted in Fig. 4 and 5. Fig. 4 depicts a typical very flexible or floppy tip 40. The tip, at the final 3-5 cm, includes a distal end 41 of the usually-Nitinol metallic core, and at the very tip 43, a smooth, elastomeric coating 42, such as a polyurethane jacket. This structure insures that the tip is very flexible.

Another embodiment is depicted in Fig. 5. This is a typical Bentson-type tip section, including a coiled-wire tip 50 made from coils 51, typically but not necessarily stainless steel material, and a smooth, atraumatic tip 53 that is laser welded or otherwise joined to coils 51. Tip section 53 is usually also made from stainless steel. However, if a radiopaque marker is desired, tip 53 may be made from a radiopaque material, such as gold or platinum. In addition, the jacket 42 may include a powder or filler of a radiopaque material, such as tungsten, so that the entire jacket or only the distal portion may be easily seen in x-rays or by using fluoroscopy. The tip section 50, as well as the entire wire guide, is preferably coated with at least one coating 54 as described above.

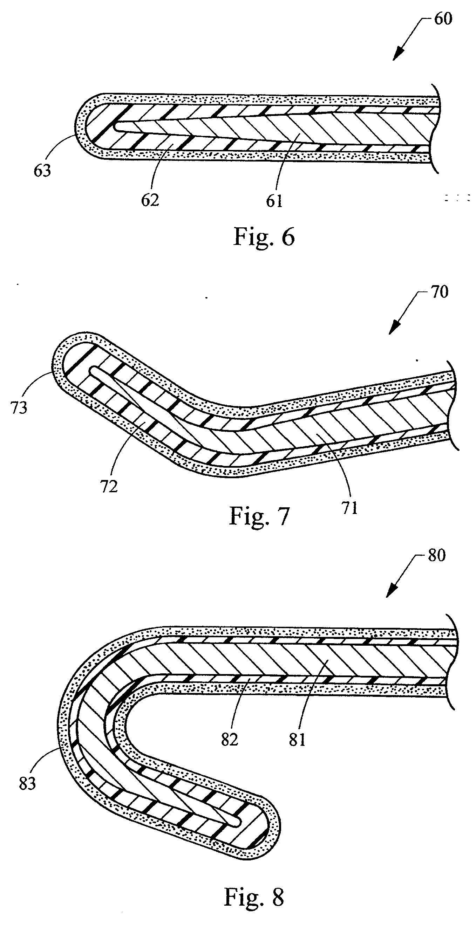

Figs. 6-10 depict additional embodiments of distal ends of flexible guide wires that may be used in any of the above embodiments. For example, Fig. 6 depicts a wire guide distal tip 60 that is straight or in-line with the remainder of the flexible wire guide, as shown in Fig. 3. Wire guide distal tip 60 may be a Bentson floppy-type tip, with an internal metallic wire 61, preferably made of Nitinol or other shape-memory alloy. Wire 61 has a straight shape because it was trained or heat-treated to assume that shape during its processing. The tip includes an outer jacket 62, such as a polyurethane jacket, and preferably includes a coating 63 as described above.

In the embodiment of Fig. 7, distal tip 70 is bent at an angle of about 45 degrees to the remainder of the guide wire. Distal tip 70 includes an inner core wire 71, which takes its angled shape because the wire has been trained to assume this shape at body temperature. Tip 70 also includes a polyurethane jacket 72 and coating 73, preferably a lubricious coating such as a hydrophilic coating, and also preferably including an underlying smooth plastic coating.

Fig. 8 depicts another embodiment of a distal tip 80 that is angled at about 180 degrees to the longitudinal axis of the guide wire. Distal tip 80 also includes core shape memory wire 81, jacket 82, and coating 83.

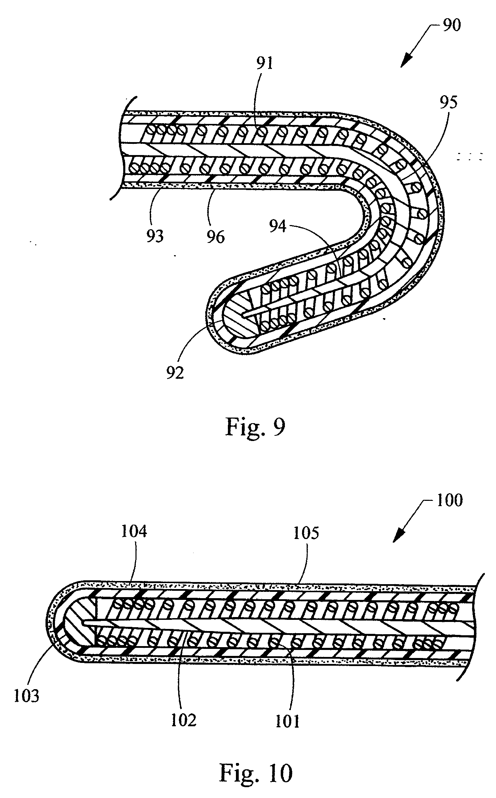

The wire guide may also be a Bentson-type wire guide with distal tips as shown in Figs. 9-10. The embodiment of Fig. 9 has been trained so that about 7 cm 95 at the distal tip 90 is bent at about 150 degrees to the longitudinal axis of the remainder of the wire guide. Distal tip 90 includes core wire 94, preferably made of Nitinol and trained to assume this shape upon reaching body temperature or other A_{f} temperature, preferably below room temperature. The wire guide includes coiled wire 91, atraumatic tip 92, and coating 93 over jacket 96, including distal tip 92.

In the embodiment of Fig. 10, distal tip 100 has been trained as a straight wire guide, including exterior coil wire 101, core wire 102, atraumatic tip 103 and coating 104 over jacket 105.

One way to manufacture the curved guide wire embodiments of Figs. 7-9 is to use a solid core mandrel or wire made of Nitinol or other shape memory alloy. The wire is first manufactured to the desired size and is then "trained" to assume a configuration with one end straight and the other end at a desired angle. One way to do this is to form the wire, as with a form or a tool at a temperature below room temperature, and then to heat treat the shape memory alloy. Typically, temperatures in the range of 400-500°C and times from 1-5 minutes are used. Other temperatures and times may also be used. Shape-memory or superelastic materials are heat treated or annealed from a weak (martinsite) structure to a strong (austenite) structure. The alloys are weak and deformable in the martinsitic state, which is thus useful for forming the wire. After transformation to the strong or martensitic state, the wire exhibits a superelastic property so long as the material remains above a transformation temperature, at which temperature it will revert to the martensitic state.

The transformation temperature is desirably a low temperature, well below the temperature of a human body, and preferably below room temperature, about 20-25°C. The transformation temperature of the wires is thus selected to be below the operating temperature of the wire, thus keeping the wire in a superelastic state. In this state, the wire advantageously returns to its original, unstressed shape when deforming stresses are removed, such as when the wire guide is removed from a package. The superelastic wire alloy also increasingly resists deformation as the stress load is increased. Thus, when a superelastic wire is deformed by being bent into a circular package for storage, the wire is placed into a state of stress. The straight end is stressed because it is bent into a circular shape with a radial dimension of the package used. The angled end is also placed into a state of stress because the angle may be partially straightened out or the angle may be exaggerated by being placed into the same package. When the wire is removed from the package, the stresses are removed, and the wire returns to its "normal" configuration of one straight end and one angled end.

The wires are formed by shaping the wires into the desired shape at room temperature or below, preferably with a cold mandrel, and then annealing the properly-shaped wire at the proper annealing temperature for a time sufficient for the transformation to a superelastic state. In one example, a wire is formed from 0.010 inch diameter Ni-Ti Nitinol wire and is annealed at 800°F (about 427°C) for about 10 minutes. The time and temperature for annealing will vary with the alloy selected and with the diameter (thickness) of the wire. The wires themselves, not merely the annealing oven, must remain at the desired temperature for the proper length of time for the annealing or heat-treatment to be complete. Proper annealing is very important for the wires to return to the desired shape during use by the surgeon or physician using the wire guide.

The heat treat operation may also be used to determine the strength and modulus of the wire. In particular, the relative stiffness or flexibility of the wire may be determined by heat-treat methods that are well known in the art. The tensile or flexural modulus may be used as a measure of the relative stiffness or flexibility of the wire, although most physicians and operating room personnel can easily detect such differences with a quick "feel" or manipulation of the finished wire guide product. A stiff wire requires more force to bend or kink, while a more flexible wire requires less force to bend or kink. The stiffness or flexibility will be most apparent in the shaft or body of the wire guide, by which is meant the intermediate portion, the distal and proximal portions of the wire guide, less the very ends or tips of the wire guide, which should remain flexible and soft out of consideration for the patient.

The "trained" wire may then be coated with a plastic suitable for use in a wire guide, as shown above for the Hi-Wire^{®} type floppy tips. By "plastic," it is meant that any polymeric material suitable for medical use with the body of a patient. For instance, the wire may be placed into a mold and polyurethane injected, poured, or cast into the mold to cover and protect the wire. Alternatively, the wire may be dipped in a coating, or placed into a tool and injection molded with polyethylene, polypropylene, or polyvinyl chloride (PVC), or other materials. Other materials and manufacturing methods may be used to manufacture wire guides with a solid wire core and a plastic covering.

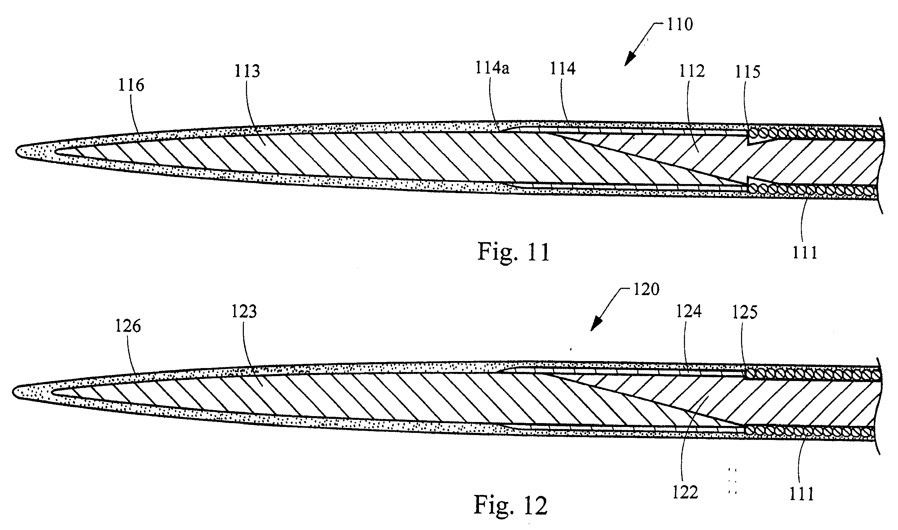

The wire guides and joining techniques described above are not the only ways to practice the invention. There are many additional embodiments, including, for example, the proximal end of a flexible wire guide may be ground to a joint or a taper for insertion into a connector. Several embodiments using this technique are disclosed in Figs. 11-14.

Fig. 11 depicts a composite wire guide 110 made up at least in part with an Amplatz wire guide 111 having an inner core 112 made of stainless steel. This portion of the Amplatz is joined to a floppy wire guide 113 which is preferably made from a shape memory alloy such as Nitinol. The ends to be joined have been ground to a bevel. The proximal end of floppy wire guide 113 is inserted into tapered connector 114 until the maximum diameter of the floppy abuts the tapered end 114a if the connector. The distal end of the Amplatz is inserted into the other end of connector 114 and then swaged in place. Amplatz inner core 112 is then laser welded to the connector at joint 115. Alternatively, a braze or solder joint may be made. The composite wire guide may also be coated with a coating 116, such as a hydrophilic coating.

Fig. 12 depicts an embodiment of a composite wire guide 120 similar to that of Fig. 11, but with no swaging. Stiffer wire guide 121 and more flexible wire guide 123 are both ground to a bevel. More flexible wire guide 123 is then inserted into nickel alloy tapered connector 124 until it is trapped. Stiffer wire guide 121 with inner core 122 is then inserted into the connector and is joined in place with joint 125. Joint 125 is preferably a laser weld, but may also be a solder or braze joint. The joint is ground smooth and the composite wire guide is then preferably coated with coating 126.

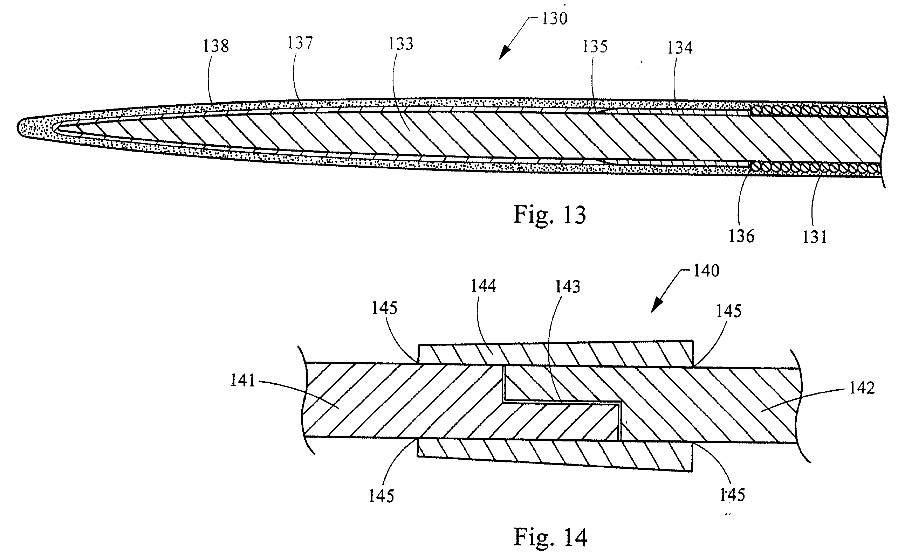

Fig. 13 depicts another possible composite wire guide 130. In this embodiment, a hollow coil type wire guide 131, such as a Hire-Wire wire guide, is joined to a more flexible Bentson floppy type wire guide 133. Flexible wire guide 133 is ground to a taper and inserted into connector 134. Joint 135 is preferably a laser weld, but may instead by a solder joint, a braze joint, or even a conventional weld joint. Second joint 136, which may be any suitable joint, joins connector 134 to flexible wire guide 133 and the end coils of coil wire guide 131. The tip includes an outer jacket 137, such as a polyurethane jacket, and preferably includes a coating 138, such as a hydrophilic coating, as described above.

The wire guide interfaces are depicted above as tapered, ground surfaces. Other interfaces may also be used, such as a plain butt joint, or as depicted in Fig. 14, a stepped joint. In Fig. 14, a joint 140 of a composite wire guide includes proximal end 141 of a floppy wire guide and the distal end 142 of a stiffer wire guide. The ends of both guides are prepared with matching steps 143. Tapered connector 144 is laser-welded at joint 145 to the wire guides. Alternatively, a braze or solder joint may be made.

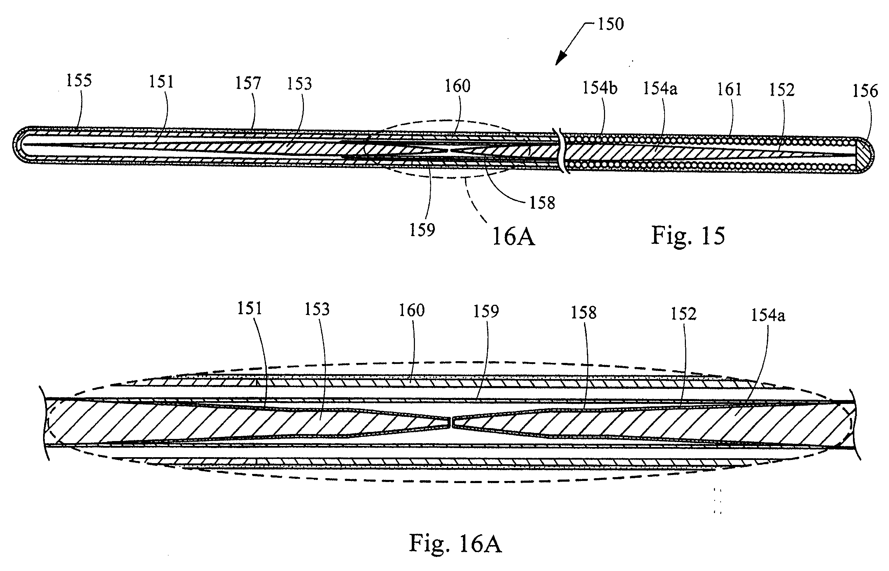

Figs. 15-16 depict yet another embodiment of a multiple-stiffness wire guide, a three-stiffness wire guide 150. Fig. 16A is an enlargement of the circled portion 16A of Fig. 15. Wire guide 150 includes a shape memory first wire guide 151 and a stainless steel second wire guide 152. The wire guides are joined at the center by a connector 159. First wire guide 151 may be made from a core 153 of a shape memory metal, such as Nitinol. Second wire guide 152 may be made from a central core 154a of stainless steel or other medically-acceptable material. Wire guides 151 and 152 are centerless ground to the desired dimension and shape. They are then soldered to a tapered central connector 159, which may be made from Nitinol. The soldering of both wire guides is thus to the Nitinol cannula. Coating 158, such as a PTFE coating, is applied to core 153.

As is well-known to those with skill in the art, first wire guide 151 is generally known as a floppy wire guide, while second wire guide 152 is generally described as a Bentson floppy. The central portion of wire guide 150 includes an outer stiff portion 160 made from coiled flat wire. The central portion is the stiffest portion of wire guide 150, and has Amplatz-type stiffness. The central outer stiff portion 160 is soldered or otherwise bonded to the central connector 159 and to wire guides 151, 152. In a preferred configuration, the central Amplatz portion 160 does not extend as far left as the left end of central connector 159, as shown in Fig. 15.

Shape-memory alloy wire guide 151 may include the central core 153 and an outer jacket 155 of polyurethane or other medically-acceptable elastomer, such as silicone. Stainless steel wire guide 152 may include a central core 154a and an outer stainless steel coil 154b which is securely affixed to a highly-polished end cap 156. Wire guide 152 also includes an outer coating 161, preferably polyethylenetetrafluoride (PTFE) or other fluoropolymer, over coiled portion 154b and end cap 156. The entire wire guide 150 also preferably includes an outer hydrophilic coating 157.

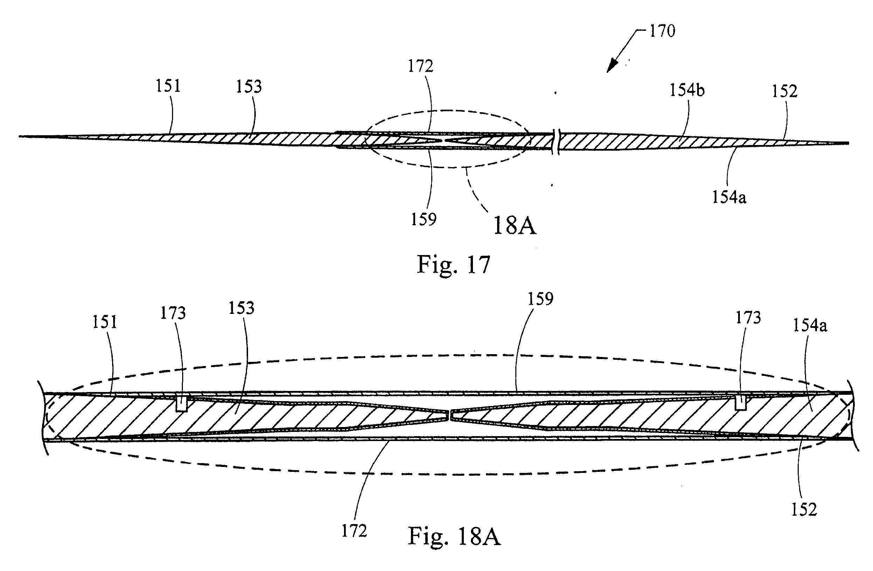

The embodiment depicted in Figs. 17-18A is similar to that depicted in Figs. 15-16. Fig. 18A is an enlargement of the circled portion 18A of Fig. 17. Three stiffness wire guide 170 includes soldering 171 that extends radially around cannula 172 and into ground and soldered key-way 173. Each of wire guides 151, 152 are ground to add slots 173 to be soldered 171 to help prevent the wire guides 151,152 from becoming disconnected from cannula 172.

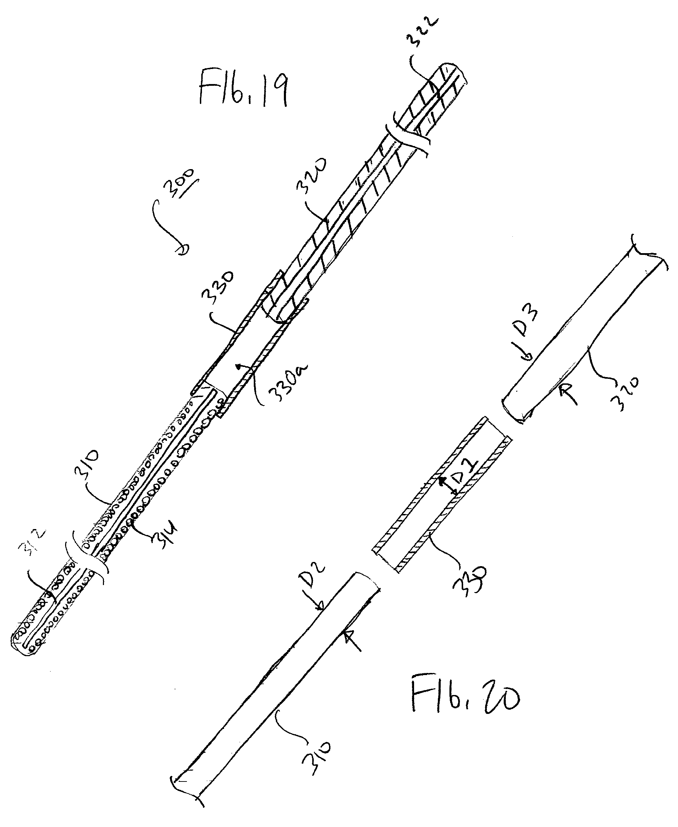

In other embodiments shown in Figs. 19-21, a composite wire guide 300 may be formed from a first end portion 310 and a second end portion 320 that are held together by a connection portion 330. The first and second end portions 310, 320 may be constructed with different materials and geometries. For example, the first end portion 310 may be constructed from a core wire 312 placed within a coiled wire 314 and function similarly to an Amplatz type wire guide. Alternatively, the first end portion 310 may be only a coiled wire and function similarly to a Bentsen type wire guide. The second end portion 320 may include an internal wire 322 formed with shape memory characteristics, such as Nitinol. Alternatively, one of the first or second end portions 310, 320 may be constructed with a core wire, a safety wire, and a coiled wire similarly to the embodiment discussed above.

The connection portion 330 may be a cannula with a cylindrical external profile and a lumen 330a formed therewithin. The connection portion 330 may be made from Nitinol, or another suitable material with shape memory characteristics. The connection portion 330 may be sized with an internal diameter D1 of the lumen 330a slightly smaller than an outer diameter D2, D3 of the proximal ends of the first and second end portions 310, 320, respectively. The connection portion 330 may be made to be sufficiently flexible to operate similarly to an Amplatz wire guide portion.

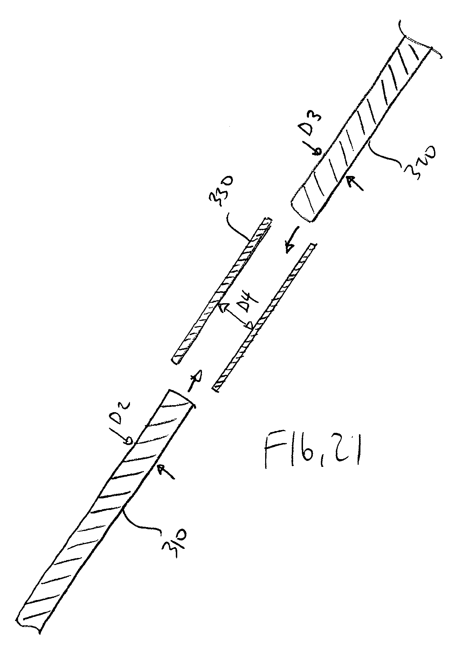

When assembling the wire guide 300, the connection portion 330 is cooled to a temperature below the Austentite Finish (Af) temperature where the material exhibits martinsitic properties. The connection portion 330 is mechanically expanded to an inner diameter D4 (FIG. 21) at least slightly larger than the outer diameter D2, D3 of the first and second end portions 310, 320, respectively. The proximal ends of the first and second end portions 310, 320 are inserted within the expanded lumen 330a of the connection portion 330, which is then heated above the material's Austenite Finish temperature and allowed to restore to its normal smaller diameter.

As the connection portion 330 warms above the Af, the connection portion 330 contracts to contact and apply compressive forces upon the proximal end portions of the first and second ends 310, 320 inserted therewithin. The compressive forces form a press fit joint between the connection portion 330 and each of the first and second end portions 310, 320. For extra strength, the first and second end portions 310, 320 may each be fixed to the connection portion 330 with a weld joint (either laser or plasma), solder joint, or with another known fixing technique. The external surfaces of the wire guide 300 may be covered with a low-friction covering, similar to the embodiments discussed above.

## Claims

1. A composite wire guide (30), comprising:
a flexible distal portion (35);
a stiff proximal portion (31); and
a tapered connector (34) joining the distal and proximal portions
**characterised in that**,
the distal portion includes a floppy tip (40).

2. The composite wire guide of Claim 1, wherein the connector (34) is tapered on at least an inner surface.

3. The composite wire guide of Claim 1, further comprising a laser weld on the tapered connector (34).

4. The composite wire guide of Claim 1, wherein the tapered connector has at least one helical or circumferential slit (37) from an inner surface to an outer surface.

5. The composite wire guide of Claim 1, wherein the connector (34) is made from a material selected from the group consisting of nickel and stainless steel.

6. The composite wire guide of Claim 1, wherein the tapered connector (34) is joined to the distal wire guide (33) by at least one of an internal wire (322) and an outer coil (314) of the distal wire guide.

7. The composite wire guide of Claim 1, wherein the floppy tip (40) is a Bentson floppy type wire guide.

8. The composite wire guide of Claim 1, wherein the laser weld between the flexible distal portion (35) and the tapered connector (34) is intermittent.

9. The composite wire guide of Claim 1, further comprising an additional wire guide connected to the stiff proximal portion.

10. The composite wire guide of claim 1, further comprising:
a central portion and;
wherein the flexible distal portion comprises a first flexible distal portion (151) made from a shape memory alloy and connected to the central portion; and
a second flexible distal portion (152) connected to the central portion,
wherein at least one of the first and second flexible distal portions are connected to the central portion by the tapered connector (159).

11. The composite wire guide of Claim 10, wherein the tapered connector has a cylindrical outer surface and an inner surface selected from the group consisting of stepped, smooth and tapered.

12. The composite wire guide of Claim 10, wherein the first flexible portion (151) and the second flexible portion (152) include a solder key-way.

13. A method of constructing a wire guide comprising:
furnishing a flexible portion with a distal end portion (310) J and a proximal end portion (320);
furnishing a tubular central portion (330) defining a lumen (330a) and made from a shape memory alloy, wherein an inner diameter of the lumen is less than an outer diameter of the proximal end portion (320);
cooling the central portion below an austenite finish temperature;
expanding a diameter of the lumen of the central portion;
inserting the proximal end portion of the flexible portion within the expanded lumen of the central portion;
heating the central portion (330) above the austenite finish temperature, and
furnishing a second flexible portion with a distal end portion and a proximal end portion, and inserting the proximal end portion of the second flexible portion within an opposite end of the lumen of the central portion from an end of the lumen that received the proximal end portion of the flexible portion.

14. The method of Claim 13, further comprising coating the composite wire guide with a hydrophilic coating.

15. The method of claim 13, further comprising applying a solder or weld joint between the flexible portion and the central portion.

## Patentansprüche

1. Zusammengesetzter Führungsdraht (30), umfassend:
einen flexiblen distalen Teil (35);
einen steifen proximalen Teil (31); und
ein konisch zulaufendes Verbindungselement (34), das den distalen und den proximalen Teil verbindet, **dadurch gekennzeichnet, dass**
der distale Teil eine flexible Spitze (40) aufweist.

2. Zusammengesetzter Führungsdraht gemäß Anspruch 1, wobei das Verbindungselement (34) an wenigstens einer Innenoberfläche konisch zulaufend ist.

3. Zusammengesetzter Führungsdraht gemäß Anspruch 1, ferner umfassend eine Laserschweißverbindung an dem konisch zulaufenden Verbindungselement (34).

4. Zusammengesetzter Führungsdraht gemäß Anspruch 1, wobei das konisch zulaufende Verbindungselement wenigstens einen helikalen oder umlaufenden Schlitz (37) von einer Innenoberfläche zu einer Außenoberfläche aufweist.

5. Zusammengesetzter Führungsdraht gemäß Anspruch 1, wobei das Verbindungselement (34) aus einem Material ausgewählt aus der Gruppe bestehend aus Nickel und rostfreiem Stahl besteht.

6. Zusammengesetzter Führungsdraht gemäß Anspruch 1, wobei das konisch zulaufende Verbindungselement (34) durch wenigstens eines von einem inneren Draht (322) und einer äußeren Wendel (314) des distalen Führungsdrahts mit dem distalen Führungsdraht (33) verbunden ist.

7. Zusammengesetzter Führungsdraht gemäß Anspruch 1, wobei die flexible Spitze (40) ein Führungsdraht von flexiblen Bentson-Typ ist.

8. Zusammengesetzter Führungsdraht gemäß Anspruch 1, wobei die Laserschweißverbindung zwischen dem flexiblen distalen Teil (35) und dem konisch zulaufenden Verbindungselement (34) intermittierend ist.

9. Zusammengesetzter Führungsdraht gemäß Anspruch 1, ferner umfassend einen zusätzlichen Führungsdraht, der mit dem steifen proximalen Teil verbunden ist.

10. Zusammengesetzter Führungsdraht gemäß Anspruch 1, ferner umfassend:
einen zentralen Teil; und
wobei der flexible distale Teil einen ersten flexiblen distalen Teil (151) bestehend aus einer Formgedächtnislegierung und verbunden mit dem zentralen Teil; und einen zweiten flexiblen distalen Teil (152) verbunden mit dem zentralen Teil umfasst,
wobei wenigstens einer von dem ersten und dem zweiten flexiblen distalen Teil durch das konisch zulaufende Verbindungselement (159) mit dem zentralen Teil verbunden ist.

11. Zusammengesetzter Führungsdraht gemäß Anspruch 10, wobei das konisch zulaufende Verbindungselement eine zylindrische Außenoberfläche und eine Innenoberfläche ausgewählt aus der Gruppe bestehend aus gestuft, glatt und konisch zulaufend aufweist.

12. Zusammengesetzter Führungsdraht gemäß Anspruch 10, wobei der erste flexible Teil (151) und der zweite flexible Teil (152) eine Löt-Keilnut umfassen.

13. Verfahren zum Herstellen eines Führungsdrahts, umfassend:
Bereitstellen eines flexiblen Teils mit einem distalen Endteil (310) und einem proximalen Endteil (320);
Bereitstellen eines rohrförmigen zentralen Teils (330), der ein Lumen (330a) definiert und aus einer Formgedächtnislegierung besteht, wobei ein Innendurchmesser des Lumens kleiner als ein Außendurchmesser des proximalen Endteils (320) ist;
Kühlen des zentralen Teils unter eine Austenit-Endtemperatur;
Expandieren eines Durchmessers des Lumens des zentralen Teils;
Einführen des proximalen Endteils des flexiblen Teils in das expandierte Lumen des zentralen Teils;
Erhitzen des zentralen Teils (330) über die Austenit-Endtemperatur; und
Bereitstellen eines zweiten flexiblen Teils mit einem distalen Endteil und einem proximalen Endteil und Einführen des proximalen Endteils des zweiten flexiblen Teils in ein dem Ende des Lumens, das den proximalen Endteil des flexiblen Teils aufgenommen hat, gegenüberliegendes Ende des Lumens des zentralen Teils.

14. Verfahren gemäß Anspruch 13, ferner umfassend Beschichten des zusammengesetzten Führungsdrahts mit einer hydrophilen Beschichtung.

15. Verfahren gemäß Anspruch 13, ferner umfassend Anbringen einer Löt- oder Schweißverbindung zwischen dem flexiblen Teil und dem zentralen Teil.

## Revendications

1. Guide-fil composite (30), comprenant :
une partie distale souple (35) ;
une partie proximale rigide (31) ; et
un connecteur effilé (34) reliant les parties distale et proximale,
**caractérisé en ce que**
la partie distale comporte une pointe lâche (40).

2. Guide-fil composite de la revendication 1, dans lequel le connecteur (34) est effilé sur au moins une surface intérieure.

3. Guide-fil composite de la revendication 1, comprenant en outre une soudure au laser sur le connecteur effilé (34).

4. Guide-fil composite de la revendication 1, dans lequel le connecteur effilé a au moins une fente hélicoïdale ou circonférentielle (37) depuis une surface intérieure jusqu'à une surface extérieure.

5. Guide-fil composite de la revendication 1, dans lequel le connecteur (34) est fabriqué à partir d'un matériau choisi dans le groupe constitué par le nickel et l'acier inoxydable.

6. Guide-fil composite de la revendication 1, dans lequel le connecteur effilé (34) est relié au guide-fil distal (33) par un fil interne (322) et/ou une bobine extérieure (314) du guide-fil distal.

7. Guide-fil composite de la revendication 1, dans lequel la pointe lâche (40) est un guide-fil Bentson de type lâche.

8. Guide-fil composite de la revendication 1, dans lequel la soudure au laser entre la partie distale souple (35) et le connecteur effilé (34) est intermittente.

9. Guide-fil composite de la revendication 1, comprenant en outre un guide-fil supplémentaire relié à la partie proximale rigide.

10. Guide-fil composite de la revendication 1, comprenant en outre :
une partie centrale ; et
dans lequel la partie distale souple comprend une première partie distale souple (151) fabriquée à partir d'un alliage à mémoire de forme et reliée à la partie centrale ; et
une deuxième partie distale souple (152) reliée à la partie centrale,
au moins une des première et deuxième parties distales souples étant reliée à la partie centrale par le connecteur effilé (159).

11. Guide-fil composite de la revendication 10, dans lequel le connecteur effilé a une surface extérieure cylindrique et une surface intérieure choisie dans le groupe constitué par à gradins, lisse et effilée.

12. Guide-fil composite de la revendication 10, dans lequel la première partie souple (151) et la deuxième partie souple (152) comportent une rainure de clavette brasée.

13. Procédé de construction d'un guide-fil comprenant :
la fourniture d'une partie souple avec une partie d'extrémité distale (310) et une partie d'extrémité proximale (320) ;
la fourniture d'une partie centrale tubulaire (330) définissant une lumière (330a) et fabriquée à partir d'un alliage à mémoire de forme, un diamètre intérieur de la lumière étant inférieur à un diamètre extérieur de la partie d'extrémité proximale (320) ;
le refroidissement de la partie centrale au-dessous d'une température de finition austénitique ;
la dilatation d'un diamètre de la lumière de la partie centrale ;
l'insertion de la partie d'extrémité proximale de la partie souple à l'intérieur de la lumière dilatée de la partie centrale ;
le chauffage de la partie centrale (330) au-dessus de la température de finition austénitique ; et
la fourniture d'une deuxième partie souple avec une partie d'extrémité distale et une partie d'extrémité proximale, et l'insertion de la partie d'extrémité proximale de la deuxième partie souple à l'intérieur d'une extrémité opposée de la lumière de la partie centrale par rapport à une extrémité de la lumière qui a reçu la partie d'extrémité proximale de la partie souple.

14. Procédé de la revendication 13, comprenant en outre le revêtement du guide-fil composite avec un revêtement hydrophile.

15. Procédé de la revendication 13, comprenant en outre l'application d'un joint brasé ou soudé entre la partie souple et la partie centrale.
